# EUROPEAN PATENT APPLICATION

(11) **EP 1 747 795 A2**
(43) Date of publication of application: **31.01.2007**
(21) Application number: 06021871.6
(22) Date of filing: 24.02.2004
(51) Int. Cl.: A61M 39/10, A61M 1/36, F16L 27/107

(54) **Extracorporeal circulation tube connector**

(30) Priority: 24.02.2003 JP 2003045352
(62) Divisional of application: 04714006.6
(71) Applicant: JMS Co., Ltd., Hiroshima-shi Hiroshima 7308652 (JP)
(72) Inventor: Katsuno, Yutaka, Akigun, Hiroshima 736-0002 (JP); Tsutsumi, Daisuke, Hiroshima-shi, Hiroshima 733-0034 (JP); Kawarabata, Shigeki, Saekigun, Hiroshima 739-0424 (JP)
(74) Representative: Grünecker, Kinkeldey, Stockmair & Schwanhäusser Anwaltssozietät

(57) **Abstract**

To realize compact packing without causing a tube kink in an extracorporeal circulation circuit of especially a pre-connecting type which uses tubes and connectors. A connector includes a first fitting member and a second fitting member into which ends of tubes are to be fitted, and a connecting member sandwiched between and connected with the first and second fitting members. The connecting member is constricted in the middle, thereby forming a thin-walled part that has a smaller wall thickness than the other parts of the connecting member which are closer to the first and second fitting members. The thin-walled part is formed as a ring around a circumference of the connecting member,

## Description

### TECHNICAL FIELD

The present invention relates to an extracorporeal circulation circuit formed by connecting tubes with connectors, and in particular relates to a preconnecting-type extracorporeal blood system.

### BACKGROUND ART

In recent years, treatment is carried out using an extracorporeal blood system that takes a patient's blood outside the body for processing such as purification and oxygenation and then return the blood to the patient. This extracorporeal blood system was first applied to hemodialyzers, and is nowadays used for pump-oxygenators, artificial livers, and plasma separators too.

For instance, cardiovascular surgery is performed while circulating a patient's blood outside the body using a pump-oxygenator system.

The pump-oxygenator system includes an artificial heart (blood pump) for pumping blood, an artificial lung for oxygenating the blood and removing carbon dioxide, and additional equipment such as a blood reservoir, a bubble remover, a heat exchanger, a blood filter, a blood draw cannula, and an arterial inflow cannula. These components of the extracorporeal blood system are connected by tubes and connectors.

Types of connectors used in the extracorporeal blood system include a straight connector for connecting two tubes and a T-connector and Y-connector for connecting three tubes (see Unexamined Japanese Patent Application Publications Nos. H11-197238, H05-15585, and H10-263076).

In general, the components of the extracorporeal blood system are each separately packed, so that assembly is necessary before use. However, such assembly requires skills and time. Besides, misconnections or contamination of components may occur during assembly. In view of these, increasing attention has been given to a preconnecting-type extracorporeal blood system. In the preconnecting-type system, the components are preconnected and the tubes are rolled up in a sterilized state, and the whole unit is housed in a package at the time of factory shipment. This being so, once the package has been delivered to a hospital or the like and opened, the extracorporeal blood system can be set up quickly without misconnections and put to use.

For the preconnecting-type extracorporeal blood system, a smaller package is preferable as it eases delivery and storage and also is likely to be discarded after use. In other words, the extracorporeal blood system needs to be packed compactly within a package of a limited size. However, when rolling up the tubes to make them compact, part of a tube near a connecting area may bend sharply and result in a kink.

When this occurs, fluids or air flowing through the tubes may gather in the kink during use. Also, the components may not be able to be positioned properly at the time of setup. In some cases, it becomes necessary to disconnect the tubes and reassemble the components.

Such a tube kink can be prevented to some extent by rolling up the tubes carefully with a great deal of time and effort when packing the system. However, it is not desirable to spend such time and effort for the packing operation.

To overcome this problem, Unexamined Japanese Patent Application Publications Nos. H11-197238 and H05-15585 propose tubes that are less prone to kink. Though these techniques can prevent a tube kink to a certain extent, the type of tube is limited and so common tubes cannot be used. Also, these techniques fail to completely suppress a tube kink near an connecting area.

### DISCLOSURE OF THE INVENTION

The present invention aims to enable an extracorporeal circulation circuit of especially a preconnecting type that uses tubes and connectors, to be packed compactly without a tube kink near a connecting area.

The stated aim can be achieved by an extracorporeal circulation tube connector including: a first fitting member having an internal space into which one end of a first tube is to be fitted; a second fitting member having an internal space into which one end of a second tube is to be fitted; and a tubular connecting member being sandwiched between and connected with the first fitting member and the second fitting member, so that an internal space of the connecting member connects with the internal space of the first fitting member and the internal space of the second fitting member, wherein the connecting member has a thin-walled part in the middle, the thin-walled part having a smaller outside diameter and a smaller wall thickness than remaining parts of the connecting member that are closer to the first fitting member and the second fitting member.

According to this construction, the wall thickness and the outside diameter of the connecting member decrease in the thin-walled part.

Such an extracorporeal circulation tube connector is more flexible than a conventional tube connector which does not have a thin-walled part. Therefore, an angle between the first fitting member and the second fitting member can be more freely varied. Hence the effect of a bending force which acts upon the tubes near the connecting area when rolling the tubes lessens.

By employing this tube connector in a preconnecting-type extracorporeal blood system, a tube kink near the connecting area can be suppressed when packing the system, with there being no need to spend much time and effort.

Unexamined Japanese Patent Application Publication No. H10-263076 discloses a technique of providing a rolling mechanism at a connector. Though this technique can suppress a kink of tubes connected with the connector, a complex manufacturing process is needed to provide the rolling mechanism at the connector. In comparison, the tube connector of the above construction can be manufactured easily, as it requires only a simple design change of constricting a connecting member of a conventional tube connector.

Also, the tube connector of the above construction can be applied to common tubes, unlike Unexamined Japanese Patent Application Publications Nos. H11-197238 and H05-15585. Here, the thin-walled part may be formed as a ring around a circumference of the connecting member.

According to this construction, the connecting member is flexible in all directions, which enhances the tube kink suppression effect.

More preferably, the tube connector may have the following constructions.

A ratio in outside diameter of the thin-walled part to the first tube and the second tube may be in a range of 0.7 to 1.3.

When the first fitting member has a greater inside diameter than the second fitting member, the outside diameter of the thin-walled part may gradually decrease from one end facing the first fitting member to another end facing the second fitting member.

The outside diameter of the thin-walled part may be substantially equal to an outside diameter of the first tube in one end facing the first fitting member, and substantially equal to an outside diameter of the second tube in another end facing the second fitting member.

An inside diameter of the connecting member may be substantially equal to an inside diameter of the first tube in one end facing the first fitting member, and substantially equal to an inside diameter of the second tube in another end facing the second fitting member.

According to this construction, the inside diameter of the connecting member is substantially equal to the inside diameters of the first and second tubes. Since there is no sudden change in diameter of the path of flow, turbulence, blood accumulation, and air accumulation can be prevented.

At least one of the first fitting member and the second fitting member may be tubular, and have a wall thickness that is greater in one end facing the connecting member than in another end.

In this case, the wall thickness of at least one of the first fitting member and the second fitting member is smaller in the edge than in the base facing the connecting member, around the whole circumference of the fitting member. This makes the edge of the fitting member more flexible while maintaining the connection with the tube, with it being possible to enhance the tube kink suppression effect.

Here, the wall thickness of the at least one of the first fitting member and the second fitting member may gradually increase from the other end to the end facing the connecting member.

According to this construction, the entire fitting member becomes flexible, which contributes to a greater tube kink suppression effect.

Here, a rate of increase in wall thickness of the at least one of the first fitting member and the second fitting member with respect to a length of the connector in a tubular direction may be in a range of 0.1 to 6.0%.

Here, a ratio in wall thickness of the other end of the at least one of the first fitting member and the second fitting member to a tube fitted into the at least one of the first fitting member and the second fitting member may be in a range of 0.15 to 0.6.

Here, the other end of the at least one of the first fitting member and the second fitting member may be in a range of 0.5 to 2.0mm in wall thickness.

Also, a ratio of a sum of lengths of the first fitting member and the second fitting member to a length of the connector in a tubular direction may be in a range of 0.5 to 0.9.

According to this construction, the tube connector can be made shorter and more flexible while maintaining the connection with the tubes, which further enhances the tube kink suppression effect.

Here, the length of the connector may be in a range of 20 to 50mm.

Also, when the connecting member, the first tube, and the second tube are each formed of a resin, the connecting member may have a substantially equal hardness to the first tube and the second tube.

Here, the connecting member, the first tube, and the second tube may each have a durometer hardness of type A according to JIS K7215 in a range of 60 to 98.

### BRIEF DESCRIPTION OF DRAWINGS

FIG. 1 is a perspective view showing tubes connected by an extracorporeal circulation tube connector to which the first embodiment of the present invention relates.
FIG. 2 is a vertical sectional view taken along the lines A-A in FIG. 1.
FIG. 3 shows an overall construction of a preconnecting-type pump-oxygenator system.
FIG. 4 shows to what degrees tubes connected by a tube connector are bent in a bend test, for an actual example corresponding to the first embodiment and a comparative example.
FIG. 5 is a perspective view and sectional view of a T-connector to which the second embodiment of the present invention relates.
FIG. 6 is a perspective view and sectional view of a Y-connector to which the second embodiment of the present invention relates.
FIG. 7 is a graph showing to what degrees tubes connected by a tube connector are bent in a bend test, for actual examples corresponding to the first and second embodiments and comparative examples.

### BEST MODE FOR CARRYING OUT THE INVENTION

Embodiments of an extracorporeal circulation tube connector of the present invention are described below, by referring to the drawings.

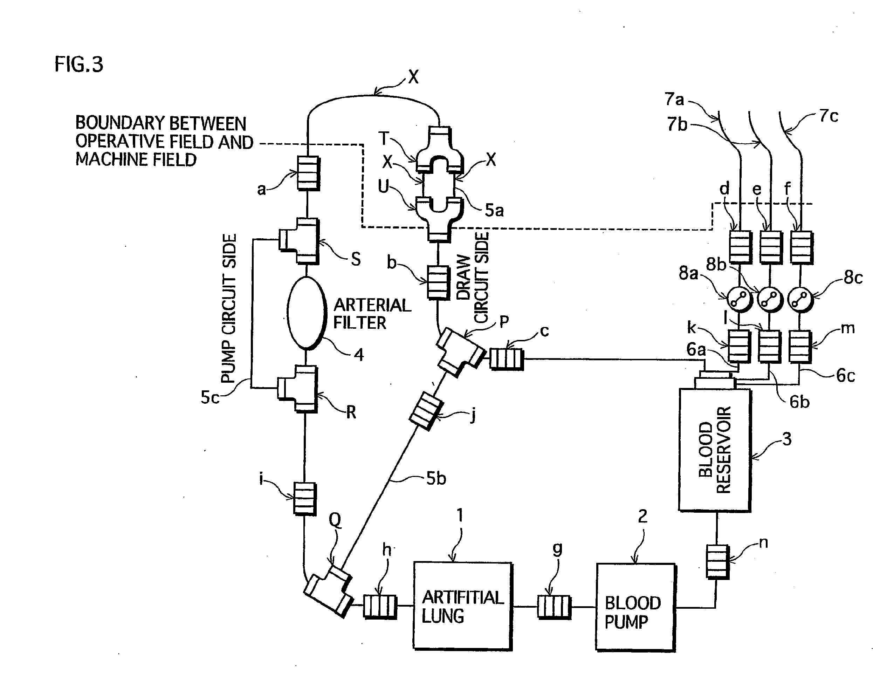

FIG. 3 shows an overall construction of a preconnecting-type pump-oxygenator system.

This preconnecting-type pump-oxygenator system includes an artificial lung 1 for oxygenating blood and removing carbon dioxide, a blood pump 2 for pumping the blood, a blood reservoir 3, an arterial filter 4, an arteriovenous circuit (cannula connection circuit) 5a, and bypass circuits 5b and 5c. The bypass circuit 5b is provided for operations such as priming and in-circuit reinfusion.

The blood reservoir 3 is connected with a vent circuit 6a and suction circuits 6b and 6c. The vent circuit 6a has a vent catheter 7a and a roller pump 8a. The suction circuits 6b and 6c have suction catheters 7b and 7c and roller pumps 8b and 8c respectively.

Circuits such as the arteriovenous circuit 5a, the bypass circuits 5b and 5c, the vent circuit 6a, and the suction circuits 6b and 6c are formed using tubes.

Straight connectors for connecting two tubes and T-connectors and Y-connectors for connecting three tubes are used in such a preconnecting-type pump-oxygenator system. In FIG. 3, straight connectors are designated by a, b, c, ... , m, and n, T-connectors are designated by P, Q, R, and S, and Y-connectors are designated by T and U.

The tubes used in the circuits have various diameters. Accordingly, some of the straight connectors are reducer-type connectors for connecting tubes of different diameters. Likewise, some of the T-connectors and Y-connectors are reducer-type connectors for connecting tubes of different diameters.

To use this preconnecting-type pump-oxygenator system, the circuitry is cut at the X position shown in FIG. 3 and is connected to instruments such as a vent catheter and a suction catheter inserted in the body of a patient.

### First Embodiment

The following describes a straight connector as an extracorporeal circulation tube connector (hereafter simply referred to as "tube connector") of the first embodiment of the present invention.

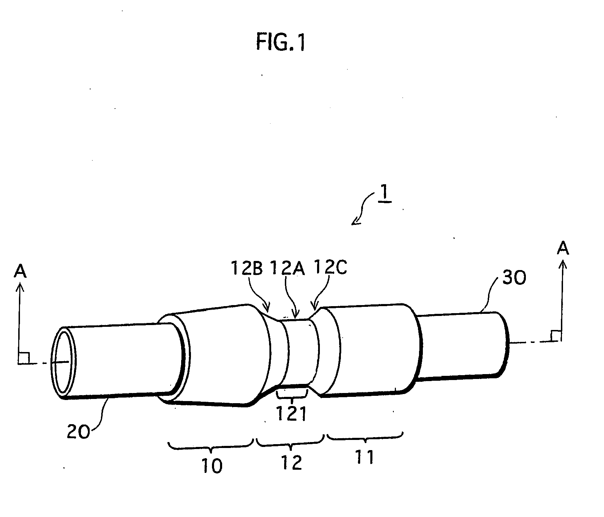

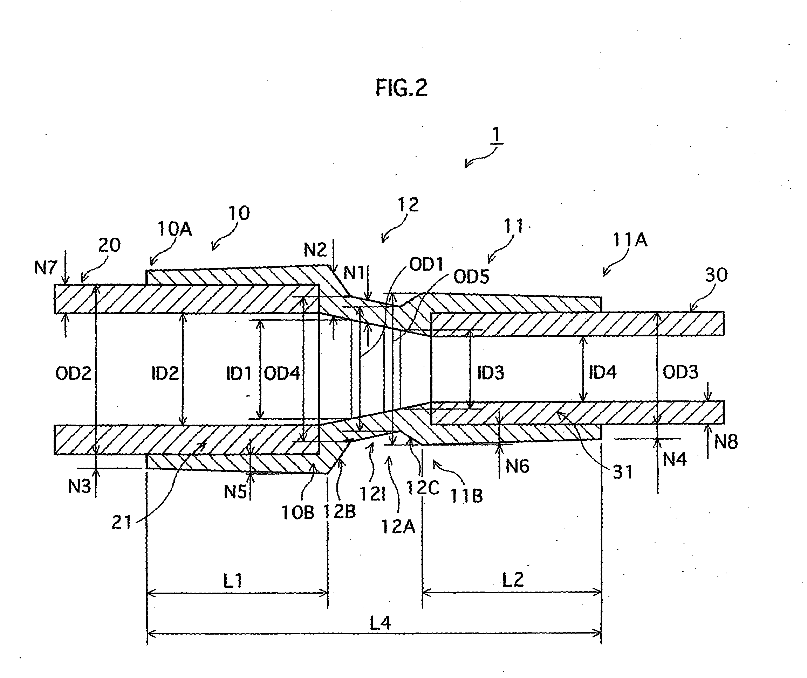

FIGS. 1 and 2 show tubes connected by a tube connector 1 in part of an extracorporeal circulation circuit such as the aforedescribed preconnecting-type pump-oxygenator system. FIG. 1 is a perspective view, whereas FIG. 2 is a vertical sectional view taken along the lines A-A in FIG. 1.

The tube connector 1 is a straight connector for connecting two tubes (a first tube 20 and a second tube 30) .

Though FIG. 2 shows the first tube 20 as having a greater diameter than the second tube 30, the first tube 20 and the second tube 30 may have the same diameter.

### (Construction of the Tube Connector 1)

The tube connector 1 is roughly made up of a first fitting member 10 into which one end 21 of the first tube 20 is to be fitted, a second fitting member 11 into which one end 31 of the second tube 30 is to be fitted, and a connecting member 12 connected between the first fitting member 10 and the second fitting member 11.

The first fitting member 10 and the second fitting member 11 are both tubular. Inside diameters of the first fitting member 10 and second fitting member 11 are substantially equal to an outside diameter OD2 of the first tube 20 and an outside diameter OD3 of the second tube 30 respectively, so that the ends 21 and 31 can be inserted deeply and connected securely. The connecting member 12 is tubular, too. An internal space of the connecting member 12 connects with internal spaces of the first fitting member 10 and the second fitting member 11.

This being so, when the first tube 20 and the second tube 30 are connected with the tube connector 1, the end 21 adjoins to one end of the connecting member 12 and the end 31 adjoins to the other end of the connecting member 12, as shown in FIG. 2. As a result, the inside of the first tube 20 and the inside of the second tube 30 connect with each other through the internal space of the connecting member 12.

An inside diameter of the connecting member 12 is substantially equal to an inside diameter ID2 of the first tube 20 at the end adjoining to the end 21, and substantially equal to an inside diameter ID4 of the second tube 30 at the end adjoining to the end 31. Therefore, if the inside diameter ID2 is equal to the inside diameter ID4, then it is preferable for the inside diameter of the connecting member 12 to be uniform from the first fitting member 10 end to the second fitting member 11 end. If the inside diameter ID2 is greater than the inside diameter ID4 as in the case of FIG. 2, on the other hand, then it is preferable for the inside diameter of the connecting member 12 to gradually decrease from the first fitting member 10 end to the second fitting member 11 end. As a result, there is no sudden change in diameter of the path of flow inside the tube connector 1 connecting the first tube 20 and the second tube 30, with it being possible to create a smooth flow without turbulence, blood accumulation, and air accumulation.

Meanwhile, an outside diameter of the connecting member 12 is smaller in a middle part 12A than in the other parts, namely, adjoining parts 12B and 12C. Here, the middle part 12A is a middle part of the connecting member 12 in a tubular direction. The adjoining part 12B is a part of the connecting member 12 that adjoins to the first fitting member 10. The adjoining part 12C is a part of the connecting member 12 that adjoins to the second fitting member 11. In other words, the connecting member 12 is constricted in the middle part 12A.

As a result, a thin-walled part 121 having a wall thickness N1 smaller than a wall thickness N2 of the adjoining parts 12B and 12C is formed in the middle part 12A of the connecting member 12.

This thin-walled part 121 is continuously formed around the whole circumference of the connecting member 12.

### (Effects of the Tube Connector 1)

As described above, the thin-walled part 121 has a smaller wall thickness and a smaller outside diameter than the adjoining parts 12B and 12C. In general, a tubular body having a smaller wall thickness and a smaller outside diameter is more flexible. Therefore, the tube connector 1 which has the thin-walled part 121 in the connecting member 12 is more flexible than a tube connector which does not have the thin-walled part 121. Which is to say, an angle between the first fitting member 10 and the second fitting member 11 can be more freely varied.

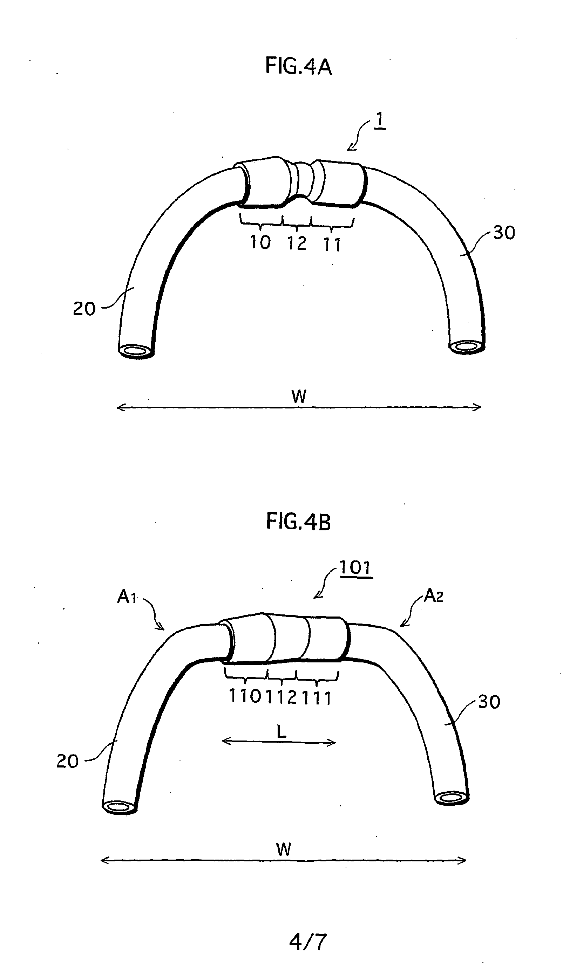

Generally, to pack a pump-oxygenator circuit in a package of a limited size, tubes which form the circuit need to be rolled up. When rolling two tubes connected by a tube connector, a bending force acts on the tubes near the connecting area, as shown in FIG. 4.

Here, if the connected tube ends are fixed straightly, the effect of the bending force on the tubes is large. If the connected tube ends can be more freely angled, on the other hand, the effect of the bending force lessens. By using the tube connector 1 to connect the first tube 20 and the second tube 30, the ends 21 and 31 can be more freely angled, with it being possible to keep the first tube 20 and the second tube 30 from kinking near the connecting area.

Thus, through the use of the tube connector 1, the tubes can be rolled up compactly without a tube kink.

If a tube kink occurs, blood may coagulate and remain in the kink, or air may gather in the kink. By using the tube connector 1, such a tube kink is suppressed and so the security of the circuit during use is improved.

A tube kink that occurs when packing an extracorporeal circulation circuit may be avoided by rolling up tubes less compactly or with a great deal of care. In actuality, however, the size of a package is limited. Also, it is not desirable to spend much time and effort for rolling the tubes. Therefore, it is necessary to roll up the tubes compactly without a tube kink in within a short time. For these reasons, the tube connector 1 has a high practical value.

### (Preferable Features of the Tube Connector 1)

### (1) Length of Each Part

The possibility of a tube kink can further be reduced by:
① making the tube connector 1 as flexible as possible, as described above; and
② making a total length L4 of the tube connector 1 in the tubular direction as small as possible, since the possibility of a tube kink is higher if the total length L4 is greater.

Regarding ②, a length L1 of the first fitting member 10 and a length L2 of the second fitting member 11 need to be at least a specified length that corresponds to an overlap width, so as to securely connect with the first tube 20 and the second tube 30. Therefore, to reduce the total length L4 of the tube connector 1, the connecting member 12 needs to bemade as short as possible while ensuring the specified lengths L1 and L2 of the first fitting member 10 and second fitting member 11.

Regarding ①, it is preferable to make the thin-walled part 121 as long as possible for greater elasticity.

To achieve both ① and ②, it is preferable to set the ratio L3/L4 in a range of 0.5 to 0.9, where L3 is a sum of the length L1 of the first fitting member 10 and the length L2 of the second fitting member 11 and L4 is the total length of the tube connector 1.

By doing so, the tube connector 1 can be made shorter and more flexible while maintaining the connection with the first tube 20 and the second tube 30. This further enhances the tube kink suppression effect.

Here, the total length L4 of the tube connector 1 is preferably in a range of 20 to 50mm.

Also, the tube connector 1 is more flexible if the thin-walled part 121 in the connecting member 12 is longer, as mentioned above. Accordingly, it is preferable to position one end of the thin-walled part 121 as close to the first fitting member 10 as possible and the other end as close to the second fitting member 11 as possible.

### (2) Outside and Inside Diameters of the Thin-walled Part 121

An outside diameter OD4 and inside diameter ID1 of the thin-walled part 121 at the first fitting member 10 end are preferably substantially equal to the outside diameter OD2 and inside diameter ID2 of the first tube 20 (OD4≒OD2, ID1≒ID2). Likewise, an outside diameter OD1 and inside diameter ID3 of the thin-walled part 121 at the second fitting member 11 end are preferably substantially equal to the outside diameter OD3 and inside diameter ID4 of the second tube 30 (OD1≒OD3, ID3≒ID4). In this way, there is no sudden change in diameter of the path of flow inside the tube connector 1 which connects the first tube 20 and the second tube 30, with it being possible to create a smooth flow of blood.

Here, the expression "substantially equal" indicates that a difference of about several mm can be tolerated (the same applies hereafter).

Also, a ratio in outside diameter of the tubes to be connected is preferably in a range of 0.7 to 1.3. Further, a ratio in outside diameter of the thin-walled part 121 to the tubes to be connected is preferably in a range of 0. 7 to 1.3. This applies not only to a straight connector but also to a T-connector and a Y-connector.

Considering a diameter of a common tube, the outside diameter OD1 of the thin-walled part 121 is preferably in a range of 4.5 to 18mm.

As shown in FIGS. 1 and 2, the wall thickness N1 of the thin-walled part 121 in the middle part 12A is approximately uniform. In other words, the inside surface and the outside surface are in parallel with each other in this part. Accordingly, not only the inside diameter but also the outside diameter of the thin-walled part 121 gradually decreases from the first fitting member 10 end to the second fitting member 11 end.

Such an approximately uniform wall thickness N1 of the thin-walled part 121 helps increase the flexibility of the connecting member 12.

### (3) Wall Thicknesses of the First Fitting Member 10 and the Second Fitting Member 11

The first fitting member 10 and the second fitting member 11 respectively have wall thicknesses N3 and N4 at their edges 10A and 11A smaller than wall thicknesses N5 and N6 at their bases 10B and 11B near the connecting member 12, around the whole circumference (N3≺N5, N4≺N6).

As a result, the edges 10A and 11A can be bent elastically to some extent while maintaining the strengths of the first fitting member 10 and second fitting member 11, when compared with the case where the wall thicknesses N3 and N4 are respectively equal to the wall thicknesses N5 and N6.

Therefore, the end 21 of the first tube 20 fitted in the first fitting member 10 and the end 31 of the second tube 30 fitted in the second fitting member 11 become more flexible near the edges 10A and 11A without losing the connection with the tube connector 1. This being so, when rolling the first tube 20 and the second tube 30, the effect of the bending force acting on the tubes near the connecting area lessens, with it being possible to prevent a tube kink near the connecting area.

Here, if the wall thicknesses of the first fitting member 10 and second fitting member 11 gradually increase from the edges 10A and 11A to the bases 10B and 11B respectively, the flexibility of the entire first fitting member 10 and second fitting member 11 increases. Accordingly, the first tube 20 and the second tube 30 are more effectively kept from kinking near the connecting area. In this case, a rate of increase in wall thickness of the first fitting member 10 and the second fitting member 11 from the edges 10A and 11A to the bases 10B and 11B with respect to the length of the tube connector 1 is preferably in a range of 0.1 to 6.0%.

Also, a ratio (N3/N7) of the wall thickness N3 of the edge 10A to the wall thickness N7 of the first tube 20 and a ratio (N4/N8) of the wall thickness N4 of the edge 11A to the wall thickness N8 of the second tube 30 are both preferably in a range of 0.15 to 0.6.

As explained above, it is desirable for both the first fitting member 10 and the second fitting member 11 to have the wall thicknesses N3 and N4 at their edges 10A and 11A smaller than the wall thicknesses N5 and N6 at their bases 10B and 11B respectively. However, even when only one of the first fitting member 10 and the second fitting member 11 has a smaller wall thickness at its edge than at its base, the effect of suppressing a tube kink near the connecting area can be achieved to a certain extent.

Also, the wall thicknesses N3 and N4 of the edges 10A and 11A are preferably in a range of 0.5 to 2.0mm. (4) Construction Material of the Tube Connector 1

A construction material of the tube connector 1 is not particularly limited, as a material used for a common tube connector is applicable. Rather, the hardness of the construction material especially in the thin-walled part 121 is important.

If the material hardness of the thin-walled part 121 is substantially equal to that of the first tube 20 and the second tube 30, the tube connector 1 can be flexibly bent when rolling the first tube 20 and the second tube 30. Hence the first tube 20 and the second tube 30 are effectively kept from kinking.

Accordingly, the same resin (e.g. polypropylen or PVC) as the one used for the first tube 20 and the second tube 30 can be used as the construction material of the tube connector 1.

Here, the tube connector 1, the first tube 20, and the second tube 30 preferably have a durometer hardness of type A according to JIS K7215 in a range of 60 to 98.

The above preferable features of the tube connector 1 in terms of (1) the length of each part, (2) the outside and inside diameters of the thin-walled part 121, (3) the wall thicknesses of the first fitting member 10 and second fitting member 11, and (4) the construction material of the tube connector 1 can be combined to enhance the tube kink suppression effect.

This embodiment describes a straight connector as one example, but the present invention is not limited to this. The present invention is equally applicable to a T-connector or Y-connector for connecting three tubes, as described below.

### Second Embodiment

The following describes a T-connector and a Y-connector as the second embodiment of the present invention.

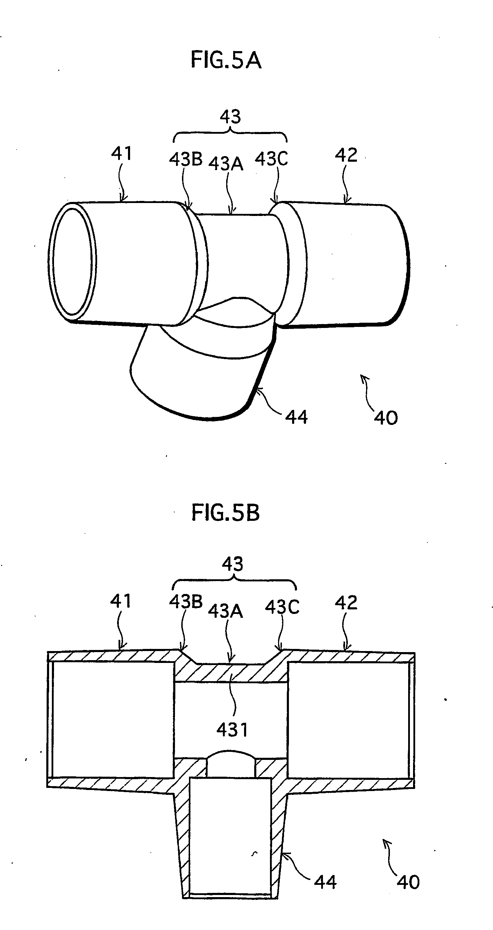

FIG. 5A is a perspective view of a T-connector 40 to which the second embodiment relates. FIG. 5B is a sectional view of the T-connector 40.

The T-connector 40 is roughly made up of a tubular first fitting member 41 into which one end of a first tube is to be fitted, a tubular second fitting member 42 into which one end of a second tube is to be fitted, a tubular connecting member 43 connected between the first fitting member 41 and the second fitting member 42, and a tubular third fitting member 44 connected vertically with the connecting member 43 and into which one end of a third tube is to be fitted. Internal spaces of the first fitting member 41, the second fitting member 42, and the third fitting member 44 each connect with an internal space of the connecting member 43.

The connecting member 43 is constricted in a middle part 43A, thereby forming a thin-walled part 431 that has a smaller wall thickness than an adjoining part 43B adjoining to the first fitting member 41 and an adjoining part 43C adjoining to the second fitting member 42.

This thin-walled part 431 is continuously formed around the whole circumference of the connecting member 43, except the area where the third fitting member 44 is connected.

Thus, the T-connector 40 has the thin-walled part 431 that has a smaller wall thickness and a smaller outside diameter than the adjoining parts 43B and 43C in the connecting member 43. Such a T-connector 40 is more flexible than a T-connector which does not have the thin-walled part 431. In other words, an angle between the first fitting member 41 and the second fitting member 42 can be more freely varied.

Through the use of this T-connector 40, the first tube and the second tube can be kept from kinking near the connecting area, as in the first embodiment.

As a result, the tubes can be rolled up compactly without a tube kink.

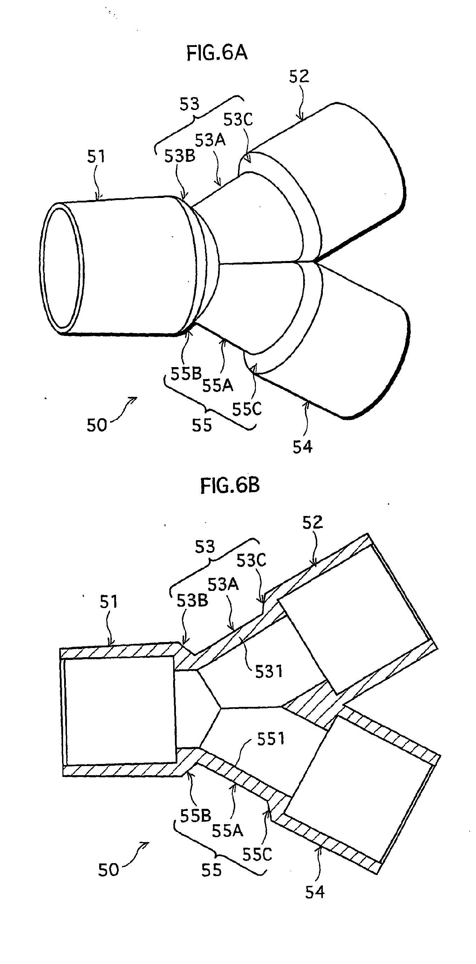

FIG. 6A is a perspective view of a Y-connector 50 to which the second embodiment relates. FIG. 6B is a sectional view of the Y-connector 50.

The Y-connector 50 is roughly made up' of a tubular first fitting member 51 into which one end of a first tube is to be fitted, a tubular second fitting member 52 into which one end of a second tube is to be fitted, a tubular third fitting member 54 into which one end of a third tube is to be fitted, a tubular first connecting member 53 connected between the first fitting member 51 and the second fitting member 52, and a tubular second connecting member 55 connected between the first fitting member 51 and the third fitting member 54. Internal spaces of the first fitting member 51 and the second fitting member 52 each connect with an internal space of the first connecting member 53. Internal spaces of the first fitting member 51 and the third fitting member 54 each connect with an internal space of the second connecting member 55. The first connecting member 53 and the second connecting member 55 are joined together on the first fitting member 51 side.

The first connecting member 53 is constricted in a middle part 53A, thereby forming a thin-walled part 531 that has a smaller wall thickness than an adjoining part 53B adjoining to the first fitting member 51 and an adjoining part 53C adjoining to the second fitting member 52. This thin-walled part 531 is continuously formed around the whole circumference of the first connecting member 53, except the area where the second connecting member 55 is connected.

Likewise, the second connecting member 55 is constricted in a middle part 55A, thereby forming a thin-walled part 551 that has a smaller wall thickness than an adjoining part 55B adjoining to the first fitting member 51 and an adjoining part 55C adjoining to the third fitting member 54. This thin-walled part 551 is continuously formed around the whole circumference of the second connecting member 55, except the area where the first connecting member 53 is connected.

Thus, the Y-connector 50 has the thin-walled part 531 that has a smaller wall thickness and a smaller outside diameter than the adjoining parts 53B and 53C in the first connecting member 53. Such a Y-connector 50 is more flexible than a Y-connector which does not have the thin-walled part 531. Also, the Y-connector 50 has the thin-walled part 551 that has a smaller wall thickness and a smaller outside diameter than the adjoining parts 55B and 55C in the second connecting member 55. Such a Y-connector 50 is more flexible than a Y-connector which does not have the thin-walled part 551.

In other words, an angle between the first fitting member 51 and the second fitting member 52 and an angle between the first fitting member 51 and the third fitting member 54 can be more freely varied.

Through the use of this Y-connector 50, the first tube, the second tube, and the third tube can be kept from kinking near the connecting area, as in the first embodiment.

As a result, the tubes can be rolled up compactly without a tube kink.

The preferable features of the tube connector 1 explained in the first embodiment in terms of (1) the length of each part, (2) the outside and inside diameters of the thin-walled part 121, (3) the wall thicknesses of the first fitting member 10 and the second fitting member 11, and (4) the construction material of the tube connector 1 also apply to the T-connector 40 and the Y-connector 50 of the second embodiment.

### Experiment on the Tube Kink Suppression Effect

(1) A comparative experiment was conducted to check the tube kink suppression effect for an actual example corresponding to the first embodiment and a comparative example.
Two short tubes were prepared as the first tube 20 and the second tube 30. The first tube 20 and the second tube 30 were connected with the tube connector 1 in the actual example, and connected with a tube connector 101 without the thin-walled part 121 in the comparative example.
The connectors and tubes used in the actual example and the comparative example are formed of PVC. The diameter of the tubes is Φ9.5mm.
The dimensions of each part of the straight connectors in the actual example and the comparative example are shown in Table 1.

**(Table 1)**

| | STRAIGHT CONNECTOR | | T-CONNECTOR | | Y-CONNECTOR | |
|---|---|---|---|---|---|---|
| | COMP. EX. | ACTUAL EX. | COMP. EX. | ACTUAL EX. | COMP. EX. | ACTUAL EX. |
| TOTAL LENGTH (mm) | 45.0 | 38.0 | 48.0 | 46.0 | 64.0 | 48.0 |
| LENGTH OF CONNECTING MEMBER (mm) | 16.0 | 9.4 | 19.0 | 17.4 | 35.0 | 19.4 |
| OUTSIDE DIAMETER OF THIN-WALLED PART (mm) | - | 14.0 | - | 14.0 | - | 14.0 |
| LENGTH OF THIN-WALLED PART (mm) | - | 8.0 | - | 16.0 | - | 13.0 |
| WALL THICKNESS OF EDGE (mm) | 2.0 | 1.0 | 2.0 | 1.0 | 2.0 | 1.0 |
| WALL THICKNESS OF BASE (mm) | 2.0 | 1.6 | 2.0 | 1.6 | 2.0 | 1.6 |
| LENGTH OF FITTING MEMBER (mm) | 14.5 | 14.3 | 14.5 | 14.3 | 14.5 | 14.3 |
| RATE OF CHANGE IN WALL THICKNESS OF FITTING MEMBER (%) | 0.0 | 3.8 | 0.0 | 3.8 | 0.0 | 3.8 |

This being so, the parts of the first tube 20 and the second tube 30 at an equal distance from both ends of the tube connector were grasped and gradually brought closer to each other so as to roll the tubes.
In both the actual example and the comparative example, no kink occurred when an interval W between the tube parts was large, but a kink occurred when the interval W was decreased. Here, an interval W1 at which a kink occurred in the actual example was smaller than an interval W2 at which a kink occurred in the comparative example.
Which is to say, when W1≺W≺W2, the first tube 20 and the second tube 30 were not kinked in the actual example as shown in FIG. 4A, but the first tube 20 and the second tube 30 were kinked at points A1 and A2 near the connecting area in the comparative example as shown in FIG. 4B.
In the comparative example, the first tube 20 and the second tube 30 were fixed straightly for a length corresponding to the length L of the tube connector 101, as shown in FIG. 4B. This caused a significant effect of the bending force on the first tube 20 and the second tube 30. In the actual example, on the other hand, the tube connector 1 is curved as shown in FIG. 4A, so that the effect of the bending force lessens.

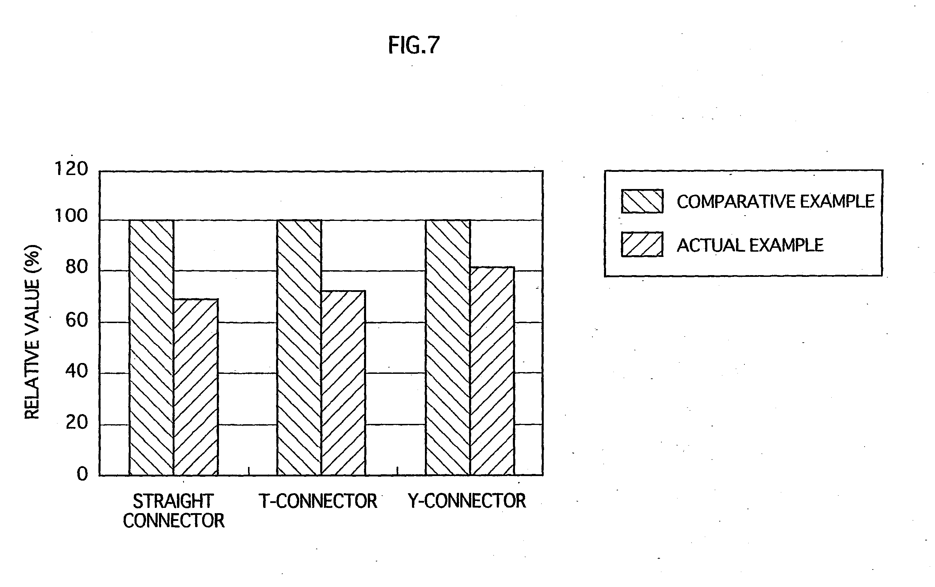

FIG. 7 shows a result of measuring the interval W at which a kink occurred as an evaluation indicator. In the drawing, the interval W is indicated by relative value.
(2) The same experiment was conducted on the T-connector 40 and the Y-connector 50 of the second embodiment as actual examples and a T-connector and a Y-connector that do not have a thin-walled part as comparative examples. The connectors and tubes used in the actual examples and the comparative examples are formed of PVC, and the diameter of the tubes is Φ9.5mm. The dimensions of each part are shown in Table 1.

FIG. 7 shows the result of this experiment, too.

As shown in FIG. 7, the interval W at which a kink occurred was smaller in the actual examples than in the comparative examples for both the T-connector and the Y-connector. These results demonstrate that the use of the tube connectors of the above embodiments makes it possible to roll up the tubes compactly without causing a tube kink.

### INDUSTRIAL APPLICABILITY

The present invention can be used for an extracorporeal blood system such as a hemodialyzer, a pump-oxygenator, an artificial liver, and a plasma separator. In particular, the present invention can be effectively used for a preconnecting-type system as it enables compact packing without causing a tube kink. The invention relates to an extracorporeal circulation tube connector comprising a first fitting member having an internal space into which one end of a first tube is to be fitted; a second fitting member having an internal space into which one end of a second tube is to be fitted; and a tubular connecting member being sandwiched between and connected with the first fitting member and the second fitting member, so that an internal space of the connecting member connects with the internal space of the first fitting member and the internal space of the second fitting member, wherein the connecting member has a thin-walled part in the middle, the thin-walled part having a smaller outside diameter and a smaller wall thickness than remaining parts of the connecting member that are closer to the first fitting member and the second fitting member.

The invention relates also to an extracorporeal circulation circuit comprising a circuit portion in which the end of the first tube and the end of the second tube are connected by the afore-described connector.

Furthermore, the invention relates to a preconnecting-type system comprising a circuit portion in which the end of the first tube and the end of the second tube are connected by the afore-described connector.

Preferably, the thin-walled part is formed as a ring around a circumference of the connecting member.

Here, it is advantageous that a ratio in outside diameter of the thin-walled part to the first tube and the second tube is in a range of 0.7 to 1.3.

It is also advantageous that the first fitting member has a greater inside diameter than the second fitting member, and the outside diameter of the thin-walled part gradually decreases from one end facing the first fitting member to another end facing the second fitting member.

Preferably, the outside diameter of the thin-walled part is substantially equal to an outside diameter of the first tube in one end facing the first fitting member, and substantially equal to an outside diameter of the second tube in another end facing the second fitting member.

Here, it is advantageous that an inside diameter of the connecting member is substantially equal to an inside diameter of the first tube in one end facing the first fitting member, and substantially equal to an inside diameter of the second tube in another end facing the second fitting member.

In a preferred embodiment, at least one of the first fitting member and the second fitting member is tubular, and has a wall thickness that is greater in one end facing the connecting member than in another end.

Here, it is advantageous that the wall thickness of the at least one of the first fitting member and the second fitting member gradually increases from the other end to the end facing the connecting member.

Preferably, a rate of increase in wall thickness of the at least one of the first fitting member and the second fitting member with respect to a length of the connector in a tubular direction is in a range of 0.1 to 6.0%.

Preferably, a ratio in wall thickness of the other end of the at least one of the first fitting member and the second fitting member to a tube fitted into the at least one of the first fitting member and the second fitting member is in a range of 0.15 to 0.6.

Here, it is preferred that the other end of the at least one of the first fitting member and the second fitting member is in a range of 0.5 to 2.0mm in wall thickness.

It is advantageous that a ratio of a sum of lengths of the first fitting member and the second fitting member to a length of the connector in a tubular direction is in a range of 0.5 to 0.9.

Here, it is preferred that the length of the connector is in a range of 20 to 50mm.

Preferably, the connecting member, the first tube, and the second tube are each formed of a resin, and the connecting member has a substantially equal hardness to the first tube and the second tube.

Preferably, the connecting member, the first tube, and the second tube each have a durometer hardness of type A according to JIS K7215 in a range of 60 to 98.

In a preferred embodiment, a third fitting member having an internal space into which one end of a third tube is to be fitted, and being connected with the connecting member so that the internal space of the connecting member connects with the internal space of the third fitting member.

Preferably, the connector further comprises a tubular additional connecting member branching off from the connecting member at one end facing the first fitting member, and a third fitting member having an internal space into which one end of a third tube is to be fitted, and being connected with the additional connecting member so that an internal space of the additional connecting member connects with the internal space of the third fitting member.

Here, it is preferred that the additional connecting member has a thin-walled part in the middle, the thin-walled part having a smaller outside diameter and a smaller wall thickness than remaining parts of the additional connecting member that are closer to the first fitting member and the third fitting member.

## Claims

1. An extracorporeal circulation tube connector (1) comprising:
- a first fitting member (10) having an internal space into which one end of a first tube (20) is to be fitted;
- a second fitting member (11) having an internal space into which one end of a second tube (30) is to be fitted; and
- a tubular connecting member (12) being sandwiched between and connected with the first fitting member (10) and the second fitting member (11), so that an internal space of the connecting member (12) connects with the internal space of the first fitting member (10) and the internal space of the second fitting member (11), wherein the connecting member (12) has a thin-walled part (121) in the middle, the thin-walled part (121) having a smaller outside diameter and a smaller wall thickness than remaining parts of the connecting member (12) that are closer to the first fitting member (10) and the second fitting member (11), and
the thin-walled part (121) is formed as a ring around a circumference of the connection member (12),
**characterized in that**
the outside diameter of the thin-walled part (121) is substantially equal to an outside diameter of the first tube (20) to be fitted in the first fitting member (10) in one end facing the first fitting member (10), and substantially equal to an outside diameter of the second tube (30) to be fitted in the second fitting member (11) in another end facing the second fitting member (11).

2. The connector of claim 1, **characterized in that** a ratio in outside diameter of the thin-walled part (121) of the first tube (20) to be fitted in the first fitting member (10) and the second tube (30) to be fitted in the second fitting member (11) is in a range of 0.7 to 1.3.

3. The connector of claim 1 or 2, **characterized in that** the first fitting member (10) has a greater inside diameter than the second fitting member (11), and the outside diameter of the thin-walled part (121) gradually decreases from one end facing the first fitting member (10) to another end facing the second fitting member (11).

4. The connector according to at least one of the preceding claims, **characterized in that** an inside diameter of the connecting member (12) is substantially equal to an inside diameter of the first tube (20) to be fitted in the first fitting member (10) in one end facing the first fitting member (10), and substantially equal to an inside diameter of the second tube (30) to be fitted in the second fitting member (11) in another end facing the second fitting member (11).

5. The connector according to at least one of the preceding claims, **characterized in that** at least one of the first fitting member (10) and the second fitting member (11) is tubular, and has a wall thickness that is greater in one end facing the connecting member (12) than in another end.

6. The connector of claim 5, **characterized in that** the wall thickness of the at least one of the first fitting member (10) and the second fitting member (11) gradually increases from the other end to the end facing the connecting member (12).

7. The connector of claim 6, **characterized in that** a rate of increase in wall thickness of the at least one of the first fitting member (10) and the second fitting member (11) with respect to a length of the connector in a tubular direction is in a range of 0.1 to 6.0%.

8. The connector of claim 5, **characterized in that** a ratio in wall thickness of the other end of the at least one of the first fitting member (10) and the second fitting member (11) to a tube (20, 30) fitted into the at least one of the first fitting member (10) and the second fitting member (11) is in a range of 0.15 to 0.6.

9. The connector of claim 8, **characterized in that** the other end of the at least one of the first fitting member (10) and the second fitting member (11) is in a range of 0.5 to 2.0 mm in wall thickness.

10. The connector according to at least one of the preceding claims, **characterized in that** a ratio of a sum of lengths of the first fitting member (10) and the second fitting member (11) to a length of the connector (1) in a tubular direction is in a range of 0.5 to 0.9.

11. The connector to at least one of the preceding claims, **characterized in that** the length of the connector (1) is in a range of 20 to 50 mm.

12. The connector to at least one of the preceding claims, **characterized in that** the connecting member (12), the first tube (20) to be fitted in the first fitting member (10), and the second tube (30) to be fitted in the second fitting member (11) are each formed of a resin, and the connecting member (12) has a substantially equal hardness to said first tube (20) and said second tube (30).

13. The connector to at least one of the preceding claims, **characterized in that** the connecting member (12), the first tube (20) to be fitted in the first fitting member (10), and the second tube (30) to be fitted in the second fitting member (11) each have a durometer hardness of type A according to JIS K7215 in a range of 60 to 98.

14. The connector to at least one of the preceding claims, **characterized in that** the connector further comprises:
a third fitting member (44) having an internal space into which one end of a third tube is to be fitted, and being connected with the connecting member (43) so that the internal space of the connecting member (43) connects with the internal space of the third fitting member (44).

15. The connector to at least one of the preceding claims, **characterized in that** the connector further comprises:
a tubular additional connecting member (55) branching off from the connecting member (53) at one end facing the first fitting member (51); and
a third fitting member (54) having an internal space into which one end of a third tube is to be fitted, and being connected with the additional connecting member (55) so that an internal space of the additional connecting member (55) connects with the internal space of the third fitting member (54).

16. The connector of claim 15, **characterized in that** the additional connecting member (55) has a thin-walled part (551) in the middle, the thin-walled part (551) having a smaller outside diameter and a smaller wall thickness than remaining parts of the additional connecting member (55) that are closer to the first fitting member (51) and the third fitting member (54).

17. An extracorporeal circulation circuit comprising a circuit portion including a connector according to at least one of the preceding claims.

18. A preconnecting-type system comprising a circuit portion including a connector according to at least one of the preceding claims.
